# EUROPÄISCHE PATENTSCHRIFT

(11) **EP 4 159 147 B1**
(45) Veröffentlichungstag und Bekanntmachung des Hinweises auf die Patenterteilung: **13.08.2025**
(21) Anmeldenummer: 21200514.4
(22) Anmeldetag: 01.10.2021
(51) Int. Cl.: A61B 18/14, A61B 5/0538, A61B 18/00, A61B 18/12

(54) **EINRICHTUNG ZUR GEWEBEBEHANDLUNG**
TISSUE TREATMENT DEVICE
DISPOSITIF DE TRAITEMENT DES TISSUS

(43) Veröffentlichungstag der Anmeldung: 05.04.2023
(73) Patentinhaber: Erbe Elektromedizin GmbH, 72072 Tübingen (DE)
(72) Erfinder: KELLER, Sandra, 72379 Hechingen (DE); SEITZ, Björn, 72793 Pfullingen (DE); HAHN, Katrin, 72622 Nürtingen (DE)
(74) Vertreter: Rüger Abel Patentanwälte PartGmbB

(56) Entgegenhaltungen:
- US-A1- 2021 038 280
- US-A1- 2021 068 895
- US-A1- 2021 153 771

## Beschreibung

Die Erfindung betrifft eine elektrochirurgische Einrichtung zur elektrothermischen Behandlung von biologischem Gewebe. Im Weiteren betrifft die Erfindung ein Verfahren zum Positionieren eines zu der Einrichtung gehörigen Instruments in dem biologischen Gewebe.

Grundsätzlich ist es bekannt, den Kontakt zwischen einer Behandlungselektrode und biologischem Gewebe durch elektrische Messungen zu erfassen und zu überwachen. Dazu offenbaren die WO 2009/065140 A1 wie auch die US 2017/0312009 A1 sowohl ein Ablationssystem als auch ein Verfahren, das in Echtzeit eine Rückmeldung über die Ausbildung einer Läsion liefert. Dazu wird der Grad der elektrischen Kopplung zwischen einer Ablationselektrode und dem biologischen Gewebe ermittelt. Auf dieser Basis lässt sich das Volumen der erzeugten Läsion abschätzen. Zur Ermittlung des Grads des Kontakts kann das Phasenverhältnis zwischen der applizierten Spannung und dem sich einstellenden Strom genutzt werden. Dazu ist ein Phasenmesskreis vorgesehen.

Die EP 2 612 612 A1 schlägt ebenfalls vor, die Phasenlage zwischen Spannung und Strom an der Behandlungselektrode eines Instruments zu erfassen. Es wird dazu die Erkenntnis ausgenutzt, dass die Phasenlagen vor Kontaktgabe, während des Kontakts sowie nach der Behandlung jeweils charakteristisch voneinander verschieden sind.

Die DE 10 2019 209 333 A1 offenbart die Bestimmung der Art eines mit einem Instrument in Berührung stehenden Gewebes. Dazu werden Wechselspannungen mit unterschiedlichen Frequenzen an das Instrument abgegeben und in das Gewebe eingeleitet und es werden die sich einstellenden Ströme überwacht. Dieses Verfahren wird auch als Impedanzspektroskopie bezeichnet.

Aus der US 2021/0068895 A1 sind ein Generator und ein zugehöriges Instrument zur Ablation von Lungentumoren bekannt. Das Instrument ist als Katheter ausgebildet und weist eine in einem Tumor einstechbare Spitze mit Elektroden auf. Der Generator beaufschlagt diese Elektroden mit einer Ablationsspannung, die geeignet ist, das Tumorgewebe zu erhitzen und dadurch abzutöten. Dabei wird die an den Elektroden gemessene Impedanz überwacht. Impedanzspitzen deuten auf Dampfblasenbildung hin. Dampfblasen isolieren jedoch die Elektroden vom Gewebe, weswegen solche Impedanzspitzen ein zumindest kurzzeitiges Abschalten des Ablationsvorgangs verursachen.

Bei der gezielten Koagulation von Gewebevolumina in lebenden menschlichen oder tierischen Patienten kommt es häufig darauf an, das entsprechende Instrument und insbesondere seine Elektroden in dem zu behandelnden insbesondere zu koagulierenden Gewebe so zu platzieren, dass ein gutes Behandlungsergebnis erreicht wird. Dabei ist die Sicht des Chirurgen auf das zu behandelnde Gewebe typischerweise eingeschränkt. Dies gilt insbesondere, wenn das zu behandelnde Gewebe nicht freigelegt ist, sondern von anderem, bei der Behandlung zu schonenden Geweben umgeben und in dieses eingebettet ist.

Daraus leitet sich die der Erfindung zugrundeliegende Aufgabe ab, eine elektrochirurgische Einrichtung anzugeben, die dem Behandler bei der Platzierung des Instruments in zu behandelndem Gewebe Unterstützung liefert.

Diese Aufgaben werden mit der elektrochirurgischen Einrichtung nach Anspruch 1 gelöst.

Zu der erfindungsgemäßen elektrochirurgischen Einrichtung gehört ein in biologisches Gewebe einstechbares Instrument mit vorzugsweise länglicher nadel- oder stiftartiger Grundform. Das Instrument weist vorzugsweise zwei oder mehrere Elektroden auf, die in axialem Abstand zueinander an dem Instrument angeordnet sind. Zum Beispiel ist das Instrument ein schlauchartiges, durch bspw. ein Endoskop in einen Körper überführbares Instrument mit einem distalen Endbereich, in dem die Elektroden als Wendelelektroden, Zylinderelektroden oder dergleichen, angeordnet sind. Das Instrument kann von innen her gekühlt sein, um eine Erwärmung der Elektroden zu unterbinden oder in Grenzen zu halten. Die vorzugsweise in axialem Abstand zueinander angeordneten Elektroden dienen vorzugsweise als Behandlungselektroden und können dazu mit einer Behandlungsspannung U_{HF} beaufschlagt werden. Dazu sind sie über entsprechende Leitungen mit einem elektrochirurgischen Generator verbunden, der dazu eingerichtet ist, eine solche Behandlungsspannung U_{HF} abzugeben. Die Behandlungsspannung U_{HF} ist eine hochfrequente Wechselspannung mit einer Frequenz typischerweise größer als 100 kHz. Darüber hinaus ist der Generator darauf eingerichtet, an die gleichen Elektroden eine Testspannung U_{T} abzugeben, die deutlich geringer ist als die Behandlungsspannung U_{HF} (bspw. Faktor 10 - 20 fach) und deswegen keinen oder nur einen sehr geringen physiologischen Effekt hat. Die Testspannung U_{T} dient zur Überprüfung und Anzeige der Positionierung des Instruments in biologischem Gewebe, beispielsweise in einem Zielgewebe, wie beispielsweise einem Tumor, der nach korrekter Positionierung des Instruments anschließend mit der Behandlungsspannung U_{HF} beaufschlagt und dadurch thermisch zerstört werden soll.

Die Erfindung macht sich die Eigenschaft zunutze, dass tumoröses Gewebe sehr gut durchblutet ist und sich dadurch von angrenzendem gesundem Gewebe elektrisch unterscheidet. Das zu der Einrichtung gehörige Instrument kann mittels der Erfindung anhand dieser Eigenschaft des Tumors in dem Tumorgewebe sicher positioniert werden. Fehlplatzierungen werden vermieden.

Der Generator weist eine Steuereinrichtung auf, die dazu eingerichtet ist, den Strom i zu erfassen, den die Testspannung U_{T} zwischen den beiden Elektroden des Instruments hervorruft. Weiter ist die Steuereinrichtung dazu eingerichtet, aus der Testspannung U_{T} und dem Strom i zwei charakteristische Größen zu ermitteln. Eine der beiden Größen hängt von der zwischen den Elektroden wirksamen Impedanz ab. Die andere der beiden Größen hängt von dem Phasenwinkel zwischen der Testspannung und dem Strom ab. Beispielsweise kann die erste Größe der Betrag der Impedanz sein während die zweite Größe der Leistungsfaktor oder eine andere von dem Phasenwinkel phi zwischen dem Strom und der Spannung abhängige Größe ist. Unter Leistungsfaktor wird der Quotient aus der zwischen den Elektroden im Gewebe umgesetzten wirksamen Wirkleistung zur Scheinleistung verstanden.

Die Steuereinrichtung überwacht die Veränderung der beiden Größen während des Einstechens des Instruments in das biologische Gewebe und prüft, ob beide Veränderungen jeweils vorgegebene Schwellwerte überschreiten. Die Veränderungen der beiden Größen kann als zeitliche Veränderung verstanden werden. Die Veränderung der jeweiligen Größe wird dann als Differenz der Größe zwischen zwei in gegebenem Zeitabstand aufeinanderfolgenden Zeitpunkten gemessen.

Die Steuereinrichtung kann auf verschiedene Weise ausgebildet sein:
In einer ersten Variante kann sie darauf eingerichtet sein, ein Signal immer dann abzugeben, wenn die Veränderungen der beiden Größen ihre beiden Schwellwerte überschreiten.
In einer anderen Variante kann die Steuereinrichtung darauf eingerichtet sein, ein erstes Signal abzugeben, wenn die Veränderungen ihre beiden Schwellwerte in einer ersten Richtung überschreiten, und ein zweites Signal abgeben, wenn die Veränderungen danach ihre Richtungen umkehren.
In einer weiteren Variante kann die Steuereinrichtung dazu eingerichtet sein, wenn beide Veränderungen jeweils vorgegebene Schwellwerte überschreiten, die beiden Größen als Bezugsgrößen festzulegen. Die Festlegung der beiden Bezugsgrößen kann optional mit der Abgabe eines ersten Signals verbunden sein, welches dem Operateur signalisiert, dass das Zielgewebe erreicht ist. Weiter kann die Steuereinrichtung darauf eingerichtet sein, ein zweites Signal abzugeben, wenn sich zwischen den beiden Größen und den Bezugsgrößen eine Differenz ergibt, die ein vorgegebenes Maß überschreitet. Alternativ kann die Steuereinrichtung darauf eingerichtet sein das zweite Signal abzugeben, wenn die Veränderungen nach Festlegung der Bezugsgrößen ihre Richtungen umkehren. Das erste Signal und das zweite Signal können gleich oder unterschiedlich sein. Es ist auch möglich, ein Signal solange zu erzeugen, wie die beiden Größen (innerhalb einer vorgegebenen oder vorgebbaren Toleranz) nicht von den beiden Bezugsgrößen abweichen.

Das abgegebene Signal kann dabei beispielsweise ein akustisches, ein optisches oder eine Kombination beider sein, wobei das optische Signal bspw. visuell auf einem Display des den elektrochirurgischen Generator enthaltenen elektrochirurgischen Systems dargestellt werden kann.

Nach dem erfindungsgemäßen Konzept sticht der Behandler das Instrument mit üblicher Geschwindigkeit in das Gewebe ein. Übliche Einstechgeschwindigkeiten liegen ungefähr bei 0,5 cm/s. Am Beispiel der Behandlung von Lungentumoren ergibt sich in gesundem Lungengewebe eine Impedanz Imp von mehr als 300 Ohm und ein Leistungsfaktor von LF = cos(phi) < 0,75. Sobald eine der beiden Elektroden, insbesondere die distale Elektrode des Instruments, in Kontakt mit Tumorgewebe kommt, verändern sich beide Größen, d.h. beispielsweise sowohl die Impedanz als auch der Leistungsfaktor. Wird das Instrument nun weiter in das Zielgewebe eingestochen und das distale Ende einer proximalen Elektrode berührt das Zielgewebe, fällt die Impedanz bei üblicher Einstechgeschwindigkeit und üblicher Instrumentengröße innerhalb einer Zeitspanne von zum Beispiel 2 Sekunden um wenigstens 60% gegenüber der zuvor gemessenen Impedanz ab während der Leistungsfaktor um beispielsweise 20% gegenüber dem Leistungsfaktor vor der festgelegten Zeitspanne (z.B. zwei Sekunden) zunimmt. Dies gilt insbesondere für übliche Instrumente mit einer in Axialrichtung gemessenen Elektrodenlänge von 8 bis 9 mm und einem axialen Elektrodenabstand von 3 bis 4 mm. Die Veränderungen der beiden Größen werden von der Steuereinrichtung erfasst. Diese kann dementsprechend ein Signal abgeben, aus dem der Behandler schließen kann, dass beide Elektroden Kontakt mit dem Tumorgewebe haben. Nach den oben genannten alternativen Ausführungsformen der Steuereinrichtung kann diese anstelle der unmittelbaren Signalabgabe oder zusätzlich dazu auch die Werte der Größen als Bezugsgrößen abspeichern und ein Signal abgeben, wenn sich die Größen gegenüber den abgespeicherten Bezugsgrößen verändern. Bspw. kann die Position der Elektroden am Instrument im Zielgewebe an einem am Gerät 15 befindlichen Display dem Anwender angezeigt werden.

Bei der Messung der beiden Größen, d.h. beispielsweise der Impedanz und des Leistungsfaktors, werden vorzugsweise jeweils mehrere aufeinanderfolgende Werte gemessen und daraus ein gleitender Mittelwert ermittelt. Die erste Größe kann beispielsweise der gleitende Mittelwert der Impedanz sein während die zweite Größe der gleitende Mittelwert des Leistungsfaktors ist. Die erfindungsgemäße Einrichtung kann dazu vorgesehen sein, die gleitenden Mittelwerte zu verschiedenen Zeitpunkten miteinander zu vergleichen und festzulegen, welche Zeitpunkte in einem gegebenen Zeitabstand zueinander gehörig sind. Damit kann der Behandler eine Anpassung an seine individuelle Einstechgeschwindigkeit vornehmen. Sticht er die Elektroden eher langsam ein, soll er einen größeren Zeitabstand wählen, sticht er das Instrument tendenziell eher schnell ein, soll er tendenziell einen kürzeren Zeitabstand wählen.

Weiter ist es möglich, den Einstechvorgang automatisiert ablaufen zu lassen, indem eine Antriebseinrichtung vorgesehen wird, die das Instrument in distaler Richtung mit bekannter, z.B. konstanter Geschwindigkeit und/oder weggesteuert bewegt. Dabei kann die Antriebseinrichtung Positionsdaten erzeugen, die die jeweilige Position des Instruments bezüglich seiner Längsrichtung kennzeichnen. In diesem Fall können die Veränderungen als zeitliche Veränderungen oder alternativ als positionsabhängige Veränderungen ermittelt werden. Letzteres ist auch möglich beim manuellen Einstechen eines Instruments, wenn dieses mit einer geeigneten Wegmesseinrichtung in Verbindung steht. Eine solche Wegmesseinrichtung kann beispielsweise Daten liefern, die anzeigen, wie weit das Instrument in distaler axialer Richtung bewegt worden ist. Im einfachsten Fall kann eine solche Wegmesseinrichtung an einem Endoskop oder Bronchoskop vorgesehen sein, in dem an dessen proximalen Ende zum Beispiel ein Messrädchen vorgesehen ist, das mit dem Instrumentenkörper (der Sonde) in Anlage steht und gedreht wird, wenn die Sonde axial vorgeschoben wird.

Weitere vorteilhafte Details der Erfindung sind Gegenstand von Unteransprüchen sowie der Beschreibung und zugehörigen Zeichnung. Es zeigen:
Figur 1 die erfindungsgemäße Einrichtung bei der Behandlung von biologischem Gewebe, in schematisierter Darstellung,
Figur 2 das zu der Einrichtung nach Figur 1 gehörige Instrument beim Einstechen in behandlungswürdiges Gewebe.
Figur 3 das Instrument nach Figur 2, in schematisierter ausschnittsweiser längsgeschnittener Darstellung,
Figur 4 Spannung und Strom an dem Instrument beim Einstechen und bei der Behandlung,
Figur 5 verschiedene aus der Spannung und dem Strom ermittelte Größen in zeitlichen Verlauf als Diagramm.

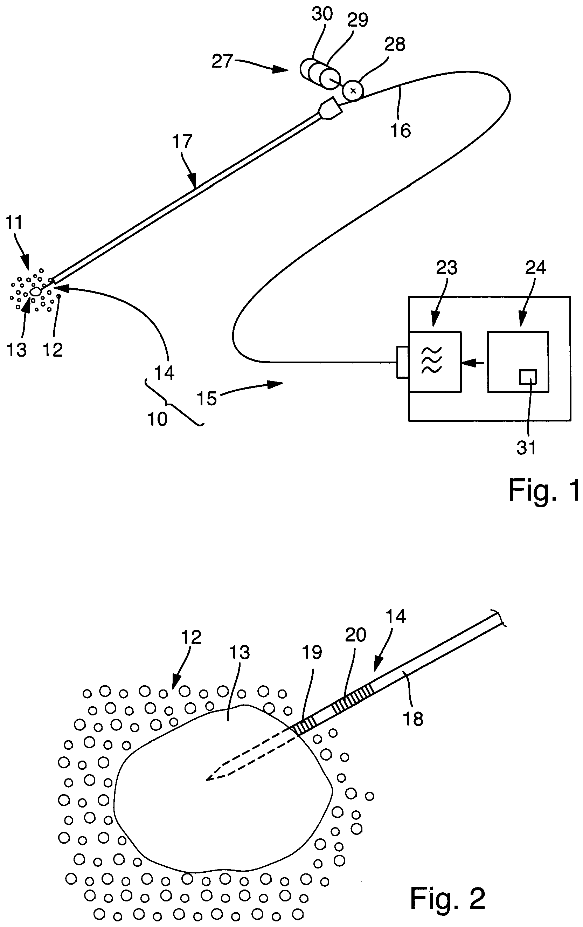

In Figur 1 ist eine Einrichtung 10 veranschaulicht, die zur Behandlung von biologischem Gewebe 11, wie beispielsweise Lungengewebe, dient, das zum Beispiel aus gesundem Lungengewebe 12 und einem darin eingebetteten Tumor 13 besteht. Die Einrichtung 10 umfasst mindestens ein Instrument 14 und ein Gerät 15 zur Speisung des Instruments 14 mit den nötigen Betriebsmedien, z.B. Kühlmedium und Strom. Das Instrument 14 kann eine flexible Sonde 16 sein, die zum Beispiel durch ein Bronchoskop 17 an das Zielgewebe 11 herangeführt wird.

Bei der Behandlung anderer Gewebe können auch andere Instrumente, zum Beispiel Endoskop, laparoskopisch anzuwendende Instrumente oder auch offenchirurgische Instrumente zur Anwendung kommen. Die Erfindung ist nicht auf die Bronchoskopie beschränkt.

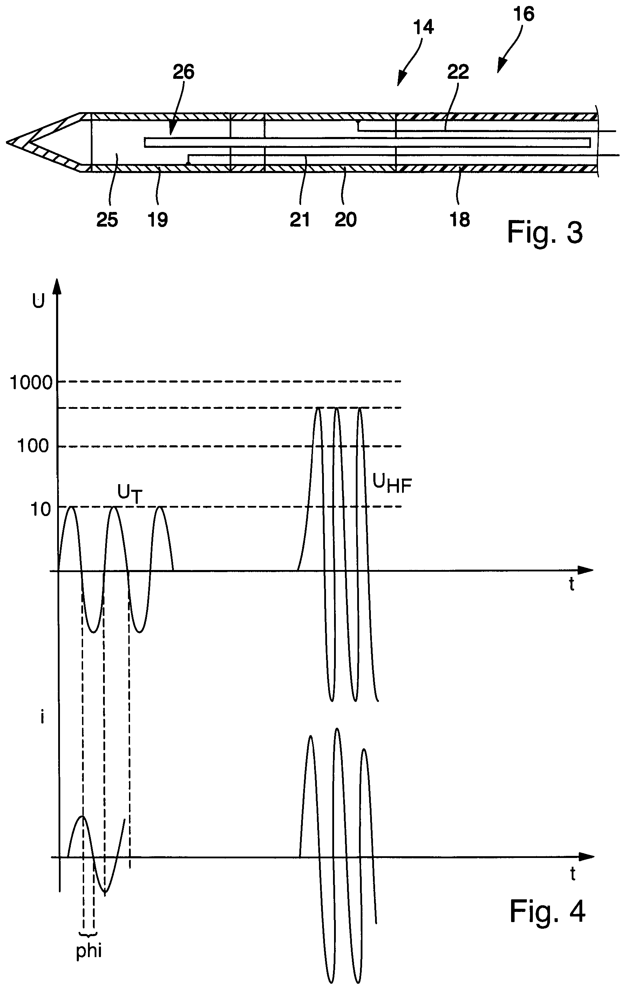

Das Instrument 14 ist in Figur 2 und 3 weiter veranschaulicht. Es weist einen länglichen, zum Beispiel als Schlauch ausgebildeten Körper 18 auf, an dem eine distale Elektrode 19 und eine in proximaler Richtung beabstandete zweiten Elektrode 20 angeordnet sind. Die Elektroden 19, 20 sind typischerweise in Axialrichtung zwischen 7 und 10 mm lang und gegeneinander elektrisch isoliert. Dazu sind sie z.B. in einem Abstand von 3 mm bis 5 mm in Axialrichtung angeordnet. Die Elektroden 19, 20 sind beispielsweise durch zylindrische Hülsen, Wendeln oder dergleichen gebildet und weisen einen Durchmesser von z.B. 2 bis 3 mm auf.

Über elektrische Leitungen, die sich vorzugsweise durch die Sonde 16 hindurch erstrecken und die an das Gerät 15 angeschlossen sind, sind die Elektroden 19, 20 mit Spannung und Strom versorgbar. Das Gerät 15 in Figur 1 weist dazu einen HF-Generator 23 auf, der von einer Steuereinrichtung 24 gesteuert wird, um entweder eine Testspannung U_{T} oder eine Behandlungsspannung U_{HF} an die Elektroden 19, 20 abzugeben.

Das Instrument 14 kann ein Lumen 25 umschließen, dem Kühlmedium über eine in dem Lumen 25 angeordnete Kapillare 26 zugeführt wird. Dieses Kühlmedium kann dazu dienen, die Elektroden 19, 20 zu kühlen, um bei der Behandlung eine Austrocknung des mit den Elektroden 19, 20 in Kontakt stehenden Gewebes zu verhindern.

Optional kann an dem Bronchoskop 17 wie in Figur 1 dargestellt zum Beispiel eine Wegmesseinrichtung 27 vorgesehen sein, mittels derer die aktuelle Position der Sonde 16 beim Einschieben in das Bronchoskop 17 und beim Einstechen in das biologische Gewebe 11 ermittelbar ist. Zu der Wegmesseinrichtung kann zum Beispiel ein mit der Sonde 16 in Reibschluss stehendes Rad 28 und ein Resolver 29 gehören. Alternativ können an dem Mantel der Sonde 16 Wegmarken angebracht sein, die von der Wegmesseinrichtung 27 ablesbar sind. Außerdem ist es möglich, die Sonde 16 automatisiert in das Gewebe 11 einzustechen. Dazu kann ein Motor 30 vorgesehen sein, der antriebsmäßig mit dem Rad 28 verbunden ist. Der Resolver 29 oder die sonstige Wegmesseinrichtung 27 und, falls vorhanden, der Motor 30 sind dann mit der Steuereinrichtung 24 verbunden.

Die Steuereinrichtung 24 kann Eingabemittel 31, wie beispielsweise eine Tastatur, einen Touchscreen oder dergleichen aufweisen, um ihren Betrieb beeinflussen zu können. Die Eingabemittel können dazu vorgesehen sein, Vorgabewerte, z.B. für Zeitdauern, Spannungen, Ströme, Leistungen, Schwellwerte und dergleichen einzugeben und an die Steuereinrichtung zu übermitteln.

Der Generator 23 ist dazu eingerichtet, eine Behandlungsspannung U_{HF} und alternativ eine in Figur 4 im Diagramm veranschaulichte Testspannung U_{T} abzugeben, die beispielsweise wenige Volt, zum Beispiel lediglich 10 Volt betragen kann. Die Testspannung U_{T} ist vorzugsweise eine Wechselspannung, insbesondere eine hochfrequente Wechselspannung mit einer Frequenz über 100 kHz. Sie wird über die Leitungen 21, 22 an die Elektroden 19, 20 übertragen.

Beim Einstechen in das Gewebe 11 fließt zwischen den Elektroden 19, 20 ein Strom i. Der Generator 23 enthält entsprechende Messmittel, um den Strom i sowohl in seiner Größe, als auch hinsichtlich seiner Phasenlage im Verhältnis zu der Testspannung U_{T} zu erfassen. Die Messmittel können beispielsweise den Phasenwinkel phi zwischen der Testspannung U_{T} und dem sich einstellenden Strom i oder eine vom Phasenwinkel phi abhängig Größe (z.B. den Leistungsfaktor) erfassen.

Der Generator 23 ist weiter dazu eingerichtet, die Behandlungsspannung U_{HF} abzugeben, die beispielsweise 100 Volt oder mehrere 100 Volt (jedoch vorzugsweise < 200 Vp) betragen kann. Der Generator 23 kann zusätzlich dazu eingerichtet sein, dabei den sich einstellenden Strom zu erfassen. Jedoch ist dies nicht zwingend.

Die Steuereinrichtung 24 ist dazu eingerichtet, beim Einstechen des Instruments 14 in das Gewebe 11 die Elektroden 19, 20 über den Generator 23 mit Testspannung zu versorgen. Dabei ist die Steuereinrichtung 24 weiter dazu eingerichtet, aus der Spannung U_{T} und dem Strom i eine erste Größe G1 zu ermitteln, die von der zwischen den Elektroden 19, 20 wirksamen Impedanz Imp abhängig ist. Diese Größe G1 kann beispielsweise die komplexe Impedanz Imp, deren Realteil R=Re{Imp}, deren Betrag |Imp| oder eine andere davon abgeleitete Größe beispielsweise der Leitwert Imp⁻¹ sein. Die erste Größe G1 kann auch durch andere von der Impedanz Imp abgeleitete Größen repräsentiert sein.

Weiter ist die Steuereinrichtung 24 dazu eingerichtet, eine zweite Größe G2 zu ermitteln, die zum Beispiel von dem Phasenwinkel phi zwischen dem Strom i und der Testspannung U_{T} abhängt. Die zweite Größe G2 kann beispielsweise der Leistungsfaktor LF = cos(phi) sein. Der Leistungsfaktor LF kann sich auch als Quotient aus der in das Gewebe 11 übertragenen Wirkleistung P und der Scheinleistung S ergeben, LF = P/S.

Weiter ist die Steuereinrichtung 24 darauf eingerichtet, die Veränderung V1 der ersten Größe G1 und die Veränderung V2 der zweiten Größe G2 zu bestimmen. Die Veränderung kann beispielsweise die zeitliche Veränderung sein. Dazu kann aus der ersten Größe G1 ein gleitender Mittelwert gebildet und die Differenz dieses Mittelwerts zwischen zwei Zeitpunkten ermittelt werden. Die beiden Zeitpunkte t1, t2 können um einen festen vorgegebenen Zeitabstand Δt beabstandet sein. Der Zeitabstand Δt kann ein zum Beispiel mittels der Eingabeeinrichtung 31 eingebbarer Zeitabstand sein. Dieser kann beispielsweise zwischen 0,1 und 5 Sekunden liegen und z.B. auf 2 Sekunden eingestellt sein. Damit ist die erste Veränderung V1 die Differenz aus der Größe G1 zu einem Zeitpunkt t2 und einem Zeitpunkt t1, die um den Zeitabstand Δt voneinander beabstandet sind.

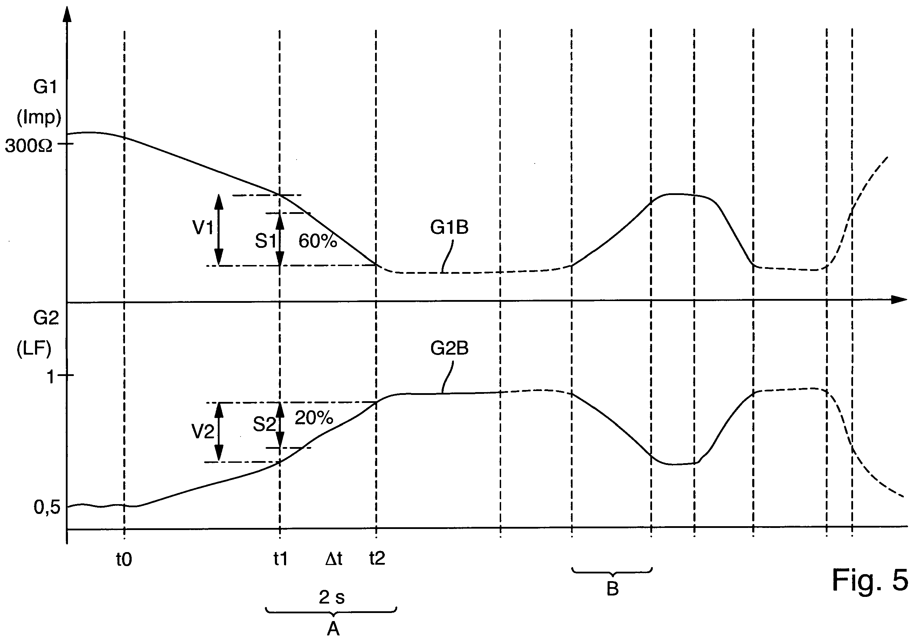

Die Verhältnisse sind in Figur 5 veranschaulicht. Ebenso kann die zweite Veränderung V2 als Differenz der zweiten Größe G2 zwischen zwei verschiedenen Zeitpunkten t1, t2 verstanden werden. Diese Differenz wird wiederum vorzugsweise zwischen gemittelten Werten der zweiten Größe G2 gebildet. Beispielsweise ist die zweite Größe G2 der gleitende Mittelwert des Leistungsfaktors. Bei diesem Beispiel wird die Differenz aus dem gleitenden Mittelwert des Leistungsfaktors LF zum Zeitpunkt t2 und dem gleitenden Mittelwert des Leistungsfaktors LF zum Zeitpunkt t1 gebildet.

Die Steuereinrichtung 24 ist darauf eingerichtet, die beiden Veränderungen V1 und V2 beim Einstechen des Instruments 14 in das Gewebe 11 zu ermitteln. In Figur 5 ist dies veranschaulicht. Zunächst befinden sich in Figur 5 ganz links beide Elektroden 19, 20 in Lungengewebe 12. Die Impedanz oder deren Betrag und somit die Größe G1 liegt bei einem relativ hohen Wert oberhalb 300 Ohm. Der Leistungsfaktor LF liegt in einem Bereich von Cosinus phi < 0,75.

Zu einem Zeitpunkt t0 beginnt die Elektrode 19 den Tumor 13 zu berühren. Bei fortgesetztem Einstechen des Instruments 14 in den Tumor 13 beginnt die Impedanz allmählich abzusinken. Zugleich steigt der Leistungsfaktor LF allmählich an. Sobald auch die zweite Elektrode 20 in den Tumor 13 eindringt (Zeitpunkt t1), beginnt die Impedanz Imp schneller abzufallen und der Leistungsfaktor LF schneller anzusteigen. Dies ist in Figur 5 in Abschnitt A durch den steileren Abfall der Kurve von G1 und den steileren Anstieg der Kurve von G2 in dem Zeitfenster Δt zu erkennen. (Es wird darauf hingewiesen, dass in Figur 5 der Verlauf der Größen G1 und G2 jeweils anhand des gleitenden Mittelwerts veranschaulicht ist, d.h. es wird zur Bestimmung der Größe G1 und der Größe G2 jeweils eine Reihe von, zum Beispiel innerhalb eines Intervalls von einer Sekunde zurückliegenden, Messwerten zu einem Mittelwert verarbeitet).

Wenn der Betrag der Differenz der gleitenden Mittelwerte von G1 in dem Intervall A, d.h. die Veränderung V1 60% überschreitet und die Veränderung V2 (d.h. der Betrag der Differenz der gleitenden Mittelwerte von G2 in dem Intervall A) 20% überschreitet, kann die Steuereinrichtung 24 je nach Gestaltung verschiedene Aktionen ausführen. Eine erste Option ist die sofortige Abgabe eines Signals, das dem Operator anzeigt, dass beide Elektroden 19, 20 ausreichend in dem Tumor 13 platziert sind. Damit ist die Behandlung des Tumors freigegeben. Eine zweite Option ist die Abgabe des Signals nach einer festen oder einstellbaren Wartezeit. Dies trägt dem Umstand Rechnung, dass die Veränderungen V1 und V2 die obigen Bedingungen meist schon erfüllen, bevor die proximale Elektrode vollständig in den Tumor eingestochen ist. Die Wartezeit von z.B. 1 s lässt den Anwender die Sonde noch etwas tiefer in den Tumor schieben. Der Behandler kann nun den Generator vom Testmode in den Behandlungsmode umschalten und somit die Behandlungsspannung an die Elektroden 19, 20 anlegen. Dies kann sowohl manuell, als auch in einer abgewandelten Ausführungsform automatisch durch die Steuereinrichtung 24 erfolgen.

In einer anderen Variante kann die Steuereinrichtung 24 auch die Größen G1 und G2 zu Ende Phase A als Bezugsgrößen G1B, G2B abspeichern und ein Signal erst dann abgeben, wenn die Größen G1, G2 anfangen, sich in Gegenrichtung zu bewegen, wie in Figur 5 in Abschnitt B dargestellt.

Die insoweit beschriebene Einrichtung 10 arbeitet wie folgt:
Nach Anschluss des Instruments 14 an das Gerät 15 wird das Instrument 14 durch das Bronchoskop 17 an das zu behandelnde Gewebe 11 herangeführt. Das Instrument 14 wird dann aus dem Bronchoskop 17 in distaler Richtung herausgeschoben und in das Gewebe 11 eingestochen. Sobald die Elektroden 19, 20 des Instruments 14 vom Anwender durch das Bronchoskop nicht mehr zu sehen sind, aktiviert der Behandler den Generator 23 im Testmode, so dass dieser die Testspannung U_{T} an die Elektroden 19, 20 des Instruments 14 abgibt. Dabei legt der Generator 23 eine geringe sinusförmige Wechselspannung von zum Beispiel weniger als 10 Vₚ (Volt Spitze) mit einer Frequenz von zum Beispiel 350 kHz bei einer Leistungsbegrenzung auf 1 Watt an das Instrument 14 an. Die Testspannung U_{T} und der daraus resultierende Strom i haben an dem Gewebe 11 aufgrund ihrer geringen Leistung keine thermische Wirkung.

Im Testmode wird das Instrument 14 im Gewebe 11 weiter vorgeschoben, wobei in festgelegten Zeitabständen (zum Beispiel im 0,1 Millisekunden Abstand) die Impedanz Imp und der Phasenwinkel phi oder daraus abgeleitete Größen G1, G2, wie z.B. der Leistungsfaktor LF, ermittelt und abgespeichert werden. Solange sich das Instrument 14 in dem Lungengewebe 12 befindet, liegt die Impedanz Imp im Bereich von oberhalb 300 Ohm und der Leistungsfaktor LF ist typischerweise geringer als 0,75.

Sobald die distale Elektrode 19 des Instruments 14 in Kontakt mit dem Tumorgewebe 13 kommt, beginnen sich sowohl die Impedanz Imp, als auch der Leistungsfaktor LF charakteristisch zu verändern. Dabei wird aber das Ablationsinstrument 14 weiterhin in das Tumorgewebe 13 eingestochen.

Wenn die Veränderung V1 des aktuellen Werts der Impedanz Imp gegenüber der Impedanz Imp vor einer gegebenen Zeitspanne (zum Beispiel Δt = 2 Sekunden) größer als ein Schwellwert S1 (z.B. 60%) ist und gleichzeitig die Veränderung V2 des aktuellen Leistungsfaktors LF gegenüber dem Leistungsfaktor LF vor 2 Sekunden größer als eine Schwelle S2 (z.B. 20%) ist, werden diese momentanen Werte der Impedanz Imp und des Leistungsfaktors LF als Tumorwerte deklariert. Die Steuereinrichtung kann dazu eingerichtet sein, die aktuellen Werte G1 und G2 als Bezugswerte G1B und G2B abzuspeichern.

Anstelle der Zeitspanne Δt von zwei Sekunden können je nach Behandler auch andere Zeitspannen eingestellt und verwendet werden. Eine Veränderung dieser Zeitspanne Δt kann durch die Eingabeeinrichtung 31 erfolgen.

Der Anwender schiebt nun das Instrument 14 weiter durch den Tumor 13 hindurch. Sobald aber von der Steuereinrichtung 24 ein Anstieg des aktuell berechneten Mittels des gleitenden Durchschnitts berechnenden Impedanzwerts und eine Verringerung des gleitenden Durchschnitts des Leistungsfaktors detektiert werden (Figur 5, Abschnitt B), gibt die Steuereinrichtung dem Anwender ein Signal, das anzeigt, dass das Instrument nun wieder Kontakt zum Lungengewebe hat. Dies bedeutet, dass sich das Instrument 14 mit seinen Elektroden 19, 20 unsymmetrisch im Tumor 13 befindet oder den Tumor 13 durchstochen hat. Der Anwender erkennt dies anhand des Signals, welches bspw. auf dem am Gerät 15 befindlichen Display angezeigt wird und kann das Ablationsinstrument zurückziehen. So findet der Anwender eine Position, die zur Behandlung geeignet ist. Die Signalgabe kann in dem Abschnitt B erfolgen, wenn die Veränderungen V1, V2 einen jeweiligen Schwellwert übersteigen. Die Schwellwerte können mit den Schwellwerten S1 und S2 identisch oder von diesen verschieden sein.

In einer abgewandelten Ausführungsform gibt die Steuereinrichtung in dem Abschnitt A (Figur 5) ein erstes und in dem Abschnitt B ein zweites Signal, so dass der Anwender die richtige Position des Instruments 14 zwischen den Abschnitten A und B findet.

Ein zur Behandlung von Lungentumoren und anderen Geweben geeignetes Instrument 14 und ein zugehöriges Gerät 15 erfassen die richtige Positionierung des Instruments 14 und seiner beiden Elektroden 19, 20 in geeignetem Zielgewebe durch Beobachtung zweier Größen G1, G2 und insbesondere deren zeitliche Veränderung. Überschreitet die Veränderung V1, V2 der beiden Größen G1, G2 jeweils gegebene Grenzwerte S1, S2, kann daraus auf die den Kontakt zwischen dem Instrument und dem zu behandelnden Gewebe und somit auch auf die Positionierung des Instruments in einer gewünschten Position geschlossen werden. Die Erfindung trägt somit wesentlich zur Erhöhung der Behandlungssicherheit bei.

### Bezugszeichen:

- 10: Einrichtung
- 11: biologisches Gewebe
- 12: gesundes Lungengewebe
- 13: Tumor
- 14: Instrument
- 15: Gerät
- 16: Sonde
- 17: Bronchoskop
- 18: Grundkörper
- 19: erste Elektrode
- 20: zweite Elektrode
- 21, 22: Leitungen
- 23: Generator
- 24: Steuereinrichtung
- 25: Lumen
- 26: Kapillare
- 27: Wegmesseinrichtung
- 28: Rad
- 29: Resolver
- 30: Motor
- 31: Eingabemittel

## Patentansprüche

1. Elektrochirurgische Einrichtung (10) zur elektrothermischen Behandlung von biologischem Gewebe (11),
mit einem in biologisches Gewebe einstechbaren Instrument (14), das einen Körper (18) mit einer ersten Elektrode (19) und mindestens einer in proximalem Abstand zu der ersten Elektrode (19) angeordneten zweiten Elektrode (20) aufweist, die mit einer hochfrequenten Testspannung (U_{T}) und mit einer hochfrequenten Behandlungsspannung (U_{HF}) versorgbar sind,
wobei die Testspannung (U_{T}) deutlich geringer ist als die Behandlungsspannung (U_{HF}) und deswegen keinen oder nur einen sehr geringen physiologischen Effekt hat,
wobei die Behandlungsspannung (U_{HF}) zur thermischen Zerstörung des biologischen Gewebes (11) geeignet ist,
mit einem Gerät (15), das einen elektrochirurgischen Generator (23) aufweist, der dazu eingerichtet ist, die Elektroden (19, 20) des Instruments (14) mit der Testspannung (U_{T}) zu speisen und dabei den sich einstellenden Strom (i) zu erfassen,
mit einer Steuereinrichtung (24), die dazu eingerichtet ist, aus der Testspannung (U_{T}) und dem Strom (i) eine erste Größe (G1) zu erfassen, die von der zwischen den Elektroden (19, 20) wirksamen Impedanz (Imp) abhängig ist, und eine zweite Größe (G2) zu erfassen, die von dem Phasenwinkel (phi) zwischen der Testspannung (U_{T}) und dem Strom (i) abhängig ist,
wobei die Steuereinrichtung (24) weiter dazu eingerichtet ist, festzustellen, ob
a) eine erste Veränderung (V1) der ersten Größe (G1) einen ersten Schwellwert (S1)
sowie
b) eine zweite Veränderung (V2) der zweiten Größe (G2) einen zweiten Schwellwert (S2) überschreiten.

2. Einrichtung nach Anspruch 1, **dadurch gekennzeichnet, dass** die erste Veränderung (V1) und die zweite Veränderung (V2) jeweils Veränderungen der zwischen verschiedenen Zeitpunkten gemessenen Größen (G1, G2) sind.

3. Einrichtung nach Anspruch 2, **dadurch gekennzeichnet, dass** die Steuereinrichtung darauf eingerichtet ist, die erste Veränderung (V1) zu bestimmen, indem die erste Größe (G1) zu verschiedenen, in einem Zeitabstand (Δt) zueinander festgelegten Zeitpunkten (t1, t2) bestimmt wird, wobei die erste Veränderung (V1) aus der Differenz der zu den verschiedenen Zeitpunkten (t2, t1) bestimmten ersten Größe (G1) ermittelt wird.

4. Einrichtung nach einem der vorstehenden Ansprüche, **dadurch gekennzeichnet, dass** die Steuereinrichtung darauf eingerichtet ist, die Größen (G1, G2) jeweils als Mittelwerte aus mehreren aufeinanderfolgenden Messungen zu bestimmen.

5. Einrichtung nach Anspruch 3 oder 4, **dadurch gekennzeichnet, dass** die Steuereinrichtung (24) mit einem Eingabemittel (31) verbunden ist, mit dem der Zeitabstand (Δt) einstellbar ist.

6. Einrichtung nach einem der vorstehenden Ansprüche,
**dadurch gekennzeichnet, dass** die Steuereinrichtung (24) darauf eingerichtet ist, den Wert der ersten und der zweiten Größe (G1, G2) als Bezugsgröße (G1B, G2B) festzulegen, wenn beide Veränderungen (V1, V2) die festgelegten Schwellwerte (S1, S2) in jeweiligen ersten Richtungen überschritten hatten.

7. Einrichtung nach Anspruch 6, **dadurch gekennzeichnet, dass** die Steuereinrichtung (24) darauf eingerichtet ist, ein Signal abzugeben, wenn sich die erste und die zweite Größe (G1, G2) ausgehend von den Bezugsgrößen (G1B, G2B) in einer jeweiligen zweiten Richtung ändern, die der jeweiligen ersten Richtung entgegengesetzt ist.

8. Einrichtung nach einem der vorstehenden Ansprüche,
**dadurch gekennzeichnet, dass** dem Instrument eine Wegmesseinrichtung (27) zugeordnet ist und dass die Steuereinrichtung (24) darauf eingerichtet ist, die Veränderungen (V1, V2) wegabhängig zu erfassen, wobei die Steuereinrichtung (24) darauf eingerichtet ist, die Veränderungen (V1, V2) durch die Differenzen zwischen in verschiedenen Instrumentenpositionen ermittelten Größen (G1, G2) zu bestimmen.

9. Einrichtung nach Anspruch 8, **dadurch gekennzeichnet, dass** die verschiedenen Instrumentenpositionen verschiedene Positionen des Instruments (14) beim Einstechen sind.

10. Einrichtung nach einem der vorstehenden Ansprüche,
**dadurch gekennzeichnet, dass** die erste Größe (G1) die Impedanz (Imp) und die zweite Größe (G2) der Leistungsfaktor (LF) ist.

11. Einrichtung nach Anspruch 10, **dadurch gekennzeichnet, dass** die erste Veränderung (V1) ein Abfall der Impedanz (Imp) um einen vorgegeben ersten Wert ist.

12. Einrichtung nach Anspruch 11, **dadurch gekennzeichnet, dass** der vorgegebene erste Wert 60% beträgt.

13. Einrichtung nach Anspruch 10 oder 11, **dadurch gekennzeichnet, dass** die zweite Veränderung (V2) ein Anstieg des Leistungsfaktors (LF) um einen vorgegeben zweiten Wert ist.

14. Einrichtung nach Anspruch 13, **dadurch gekennzeichnet, dass** der vorgegebene zweite Wert 20% beträgt.

## Claims

1. An electrosurgical device (10) for the electrothermal treatment of biological tissue (11),
having an instrument (14) that can be penetrated into biological tissue and that has a body (18) with a first electrode (19) and at least one second electrode (20) arranged at a proximal distance from the first electrode (19), which can be supplied with a radio frequency test voltage (U_{T}) and with a radio frequency treatment voltage (U_{HF}),
wherein the test voltage (U_{T}) is significantly lower than the treatment voltage (U_{HF}) and therefore has no or only a very low physiological effect,
wherein the treatment voltage (U_{HF}) is suitable for the thermal destruction of the biological tissue (11),
having an apparatus (15) comprising an electrosurgical generator (23) that is configured to supply the electrodes (19, 20) of the instrument (14) with the test voltage (U_{T}) and to detect the resulting current (i) thereby,
having a control device (24) that is configured to detect from the test voltage (U_{T}) and the current (i) a first parameter (G1) that is dependent on the effective impedance (Imp) between the electrodes (19, 20) and a second parameter (G2) that is dependent on the phase angle (phi) between the test voltage (U_{T}) and the current (i),
wherein the control device (24) is further configured to determine whether
a) a first change (V1) in the first parameter (G1) exceeds a first threshold value (S1)
and
b) a second change (V2) in the second parameter (G2) exceeds a second threshold value (S2).

2. The device according to claim 1, **characterized in that** the first change (V1) and the second change (V2) are in each case changes in the parameters (G1, G2) measured between different points in time.

3. The device according to claim 2, **characterized in that** the control device is configured to determine the first change (V1) by determining the first parameter (G1) at different points in time (t1, t2) defined at a time interval (Δt) from one another, wherein the first change (V1) is determined from the difference of the first parameter (G1) determined at the different points in time (t1, t2).

4. The device according to any of the preceding claims,
**characterized in that** the control device is configured to determine the parameters (G1, G2) in each case as averages from several successive measurements.

5. The device according to claim 3 or 4, **characterized in that** the control device (24) is connected with an input means (31) by means of which the time interval (Δt) can be adjusted.

6. The device according to any of the preceding claims,
**characterized in that** the control device is configured to set the value of the first and the second parameter (G1, G2) as reference parameter (G1B, G2B) if both changes (V1, V2) have exceeded the defined threshold values (S1, S2) in respective first directions.

7. The device according to claim 6, **characterized in that** the control device (24) is configured to output a signal, if the first and the second parameter (G1, G2) change in a respective second direction, which is opposite to the respective first direction, starting from the reference parameters (G1B, G2B).

8. The device according to any of the preceding claims,
**characterized in that** a displacement measurement device (27) is assigned to the instrument and that the control device (24) is configured to detect the changes (V1, V2) as a function of displacement, wherein the control device (24) is configured to determine the changes (V1, V2) by the differences between parameters (G1, G2) determined in different instrument positions.

9. The device according to claim 8, **characterized in that** the different instrument positions are different positions of the instrument (14) during penetration.

10. The device according to any of the preceding claims,
**characterized in that** the first parameter (G1) is the impedance (Imp) and the second parameter (G2) is the power factor (LF).

11. The device according to claim 10, **characterized in that** the first change (V1) is a drop in the impedance (Imp) by a predetermined first value.

12. The device according to claim 11, **characterized in that** the predetermined first value is 60%.

13. The device according to claim 10 or 11, **characterized in that** the second change (V2) is an increase in the power factor (LF) by a predetermined second value.

14. The device according to claim 13, **characterized in that** the predetermined second value is 20%.

## Revendications

1. Dispositif électrochirurgical (10) destiné au traitement électrothermique de tissus biologiques (11),
comprenant un instrument (14) qui peut être piqué dans des tissus biologiques et qui présente un corps (18) comportant une première électrode (19) et au moins une deuxième électrode (20) disposée à distance proximale de la première électrode (19), qui peuvent être alimentées avec une tension d'essai (U_{T}) à haute fréquence et avec une tension de traitement (U_{HF}) à haute fréquence,
dans lequel la tension d'essai (U_{T}) est nettement plus faible que la tension de traitement (U_{HF}) et n'a pour cette raison pas d'effet physiologique ou seulement un effet physiologique très faible,
dans lequel la tension de traitement (U_{HF}) convient à la destruction thermique des tissus biologiques (11),
comprenant un appareil (15) qui présente un générateur électrochirurgical (23) qui est conçu pour alimenter les électrodes (19, 20) de l'instrument (14) avec la tension d'essai (U_{T}) en mesurant le courant (i) qui s'installe de ce fait,
comprenant un dispositif de commande (24) qui est conçu pour recueillir, à partir de la tension d'essai (U_{T}) et du courant (i), une première grandeur (G1) qui est fonction de l'impédance (Imp) effective entre les électrodes (19, 20), et une deuxième grandeur (G2) qui est fonction de l'angle de phase (phi) entre la tension d'essai (U_{T}) et le courant (i),
le dispositif de commande (24) étant par ailleurs conçu pour déterminer si
a) une première variation (V1) de la première grandeur (G1) dépasse une première valeur seuil (S1)
et
b) une deuxième variation (V2) de la deuxième grandeur (G2) dépasse une deuxième valeur seuil (S2).

2. Dispositif selon la revendication 1, **caractérisé en ce que** la première variation (V1) et la deuxième variation (V2) sont respectivement des variations des grandeurs (G1, G2) mesurées entre des instants différents.

3. Dispositif selon la revendication 2, **caractérisé en ce que** le dispositif de commande est conçu pour déterminer la première variation (V1), en déterminant la première grandeur (G1) à des instants (t1, t2) différents fixés avec un intervalle de temps (Δt) l'un par rapport à l'autre, la première variation (V1) étant calculée à partir de la différence de la première grandeur (G1) déterminée aux instants (t2, t1) différents.

4. Dispositif selon une des revendications précédentes, **caractérisé en ce que** le dispositif de commande est conçu pour déterminer les grandeurs (G1, G2) respectivement en tant que valeurs moyennes à partir de plusieurs mesures successives.

5. Dispositif selon la revendication 3 ou 4, **caractérisé en ce que** le dispositif de commande (24) est relié à un moyen de saisie (31) qui permet de régler l'intervalle de temps (Δt).

6. Dispositif selon une des revendications précédentes, **caractérisé en ce que** le dispositif de commande (24) est conçu pour définir la valeur de la première et de la deuxième grandeur (G1, G2) en tant que grandeur de référence (G1B, G2B), lorsque les deux variations (V1, V2) ont dépassé les valeurs seuils (S1, S2) définies dans des premières directions respectives.

7. Dispositif selon la revendication 6, **caractérisé en ce que** le dispositif de commande (24) est conçu pour émettre un signal lorsque, partant des grandeurs de référence (G1B, G2B), la première et la deuxième grandeur (G1, G2) varient dans une deuxième direction respective qui est orientée à l'opposé de la première direction respective.

8. Dispositif selon une des revendications précédentes, **caractérisé en ce qu'**un dispositif de mesure de déplacement (27) est associé à l'instrument, et **en ce que** le dispositif de commande (24) est conçu pour recueillir les variations (V1, V2) en fonction du déplacement, le dispositif de commande (24) étant conçu pour déterminer les variations (V1, V2) sur la base des différences entre des grandeurs (G1, G2) déterminées dans des positions d'instrument différentes.

9. Dispositif selon la revendication 8, **caractérisé en ce que** les différentes positions d'instrument sont des positions différentes de l'instrument (14) lors du piquage.

10. Dispositif selon une des revendications précédentes, **caractérisé en ce que** la première grandeur (G1) est l'impédance (Imp) et la deuxième grandeur (G2) est le facteur de puissance (LF).

11. Dispositif selon la revendication 10, **caractérisé en ce que** la première variation (V1) est une baisse de l'impédance (Imp) d'une première valeur prédéfinie.

12. Dispositif selon la revendication 11, **caractérisé en ce que** la première valeur prédéfinie est de 60 %.

13. Dispositif selon la revendication 10 ou 11, **caractérisé en ce que** la deuxième variation (V2) est une augmentation du facteur de puissance (LF) d'une deuxième valeur prédéfinie.

14. Dispositif selon la revendication 13, **caractérisé en ce que** la deuxième valeur prédéfinie est de 20 %.
